# FASCICULE DE BREVET EUROPEEN

(11) **EP 0 511 118 B1**
(45) Date de publication et mention de la délivrance du brevet: **01.03.1995**
(21) Numéro de dépôt: 92401189.3
(22) Date de dépôt: 24.04.1992
(51) Int. Cl.: A61K 7/48, A61K 7/00

(54) **Utilisation du pyrrolidone carboxylate de lysine et/ou d'arginine comme substance antioxydante dans une composition cosmétique ou pharmaceutique**
Verwendung von Lysin und/oder Arginin Pyrrolidone-Carboxylate als Antioxidationsmittel in Kosmetika oder Pharmazeutika
Use of lysine and or arginine pyrrolidone carboxylate as anti-oxydant agent in a cosmetic or pharmaceutical composition

(30) Priorité: 24.04.1991 FR 9105062
(43) Date de publication de la demande: 28.10.1992
(73) Titulaire: L'OREAL, 75008 Paris (FR)
(72) Inventeur: N'Guyen, Lan Quang, F-92160 Antony (FR); Soudant, Etienne, F-94260 Fresnes (FR)
(74) Mandataire: Stalla-Bourdillon, Bernard

(56) Documents cités:
- PATENT ABSTRACTS OF JAPAN, vol. 10, no. 182 (C-356)[2238], 25 juin 1986;& JP-A-61 30 509 (SHISEIDO CO., LTD) 12-02-1986
- PARFUMS, COSMETIQUES, AROMES, no. 93, juin-juillet 1990, pages 93-99, Paris,FR; M. DESRAME et al.: "L'acide pyrrolidone carboxylique et ses dérivés dansl'industrie cosmétique"
- PATENT ABSTRACTS OF JAPAN, vol. 5, no. 136 (C-69[808], 28 août 1981;& JP-A-56 71 020 (SHISEIDO) 13-06-1981

## Description

La présente invention a pour objet l'utilisation du pyrrolidone carboxylate de lysine (ci-après pidolate de lysine) et/ou du pyrrolidone carboxylate d'arginine (ci-après pidolate d'arginine) comme substance antioxydante dans la préparation d'une composition cosmétique ou pharmaceutique ainsi que l'utilisation du pidolate de lysine ou du pidolate d'arginine associé à au moins un dérivé phénolique.

On sait que les corps gras ont tendance à s'oxyder, même à température ambiante et cette oxydation (ou rancissement) leur fait acquérir de nouvelles propriétés, notamment gustatives ou olfactives qui sont généralement considérées comme indésirables lorsque ces corps gras sont incorporés, par exemple, dans des compositions alimentaires ou dans des compositions cosmétiques.

Afin de prévenir l'oxydation, les compositions à base de corps gras contiennent des agents protecteurs ou agents antioxydants.

Parmi les antioxydants connus, on peut notamment citer l'acide ascorbique qui agit notamment par absorption directe d'oxygène. L'acide ascorbique présente toutefois l'inconvénient d'être très peu soluble dans les corps gras.

Afin de remédier à cet inconvénient de l'acide ascorbique, il a également été proposé l'utilisation de divers esters d'ascorbyle tels que par exemple le stéarate, le palmitate ou le laurate d'ascorbyle.

Par ailleurs, on sait qu'en dehors de leurs propriétés antioxydantes propres, les dérivés ascorbiques ont également la propriété d'améliorer l'activité d'agents antioxydants tels que les tocophérols ou l'acide caféique et ses esters en favorisant la régénération de ces agents antioxydants.

On a également proposé diverses améliorations de ces agents antioxydants binaires du type dérivés ascorbiques + tocophérols ou dérivés ascorbiques + dérivés caféiques, en prévoyant l'addition d'un troisième constituant améliorant encore les effets antioxydants. Parmi les troisièmes constituants de ces systèmes ternaires, on peut notamment citer l'acide p-aminobenzoïque, des phospholipides ou encore des amines.

Il est par ailleurs connu que certains acides aminés possèdent des propriétés antioxydantes, AHMAD M.M., JAOCS, 60(4), 1983, pp.837-840. Les propriétés antioxydantes de ces acides aminés peuvent notamment être renforcées par l'association avec diverses autres substances antioxydantes telles que par exemples l' -tocophérol.

Après diverses études, on a maintenant constaté qu'il était possible d'obtenir une excellente activité antioxydante dans des compositions cosmétiques en utilisant comme substance active du pidolate de lysine ou du pidolate d'arginine.

Le pidolate de lysine ainsi que le pidolate d'arginine ont été cités dans la littérature comme constituant des agents hydratants mais n'ont jamais été décrits comme ayant des propriétés antioxydantes.

La présente invention a donc pour objet l'utilisation du pidolate de lysine ou du pidolate d'arginine, comme substance antioxydante dans la préparation d'une composition cosmétique ou pharmaceutique destinée au traitement de la peau et en particulier de son vieillissement.

Le pidolate d'arginine ainsi que le pidolate de lysine sont comme indiqués ci-dessus des produits connus qui ont été décrits par ZANOTTI, Prodotto Chimico, Marzo 1982, pp. 25-28.

Selon l'invention, le pidolate d'arginine et/ou de lysine est utilisé dans les compositions cosmétiques à une concentration comprise entre 0,1 et 20 % en poids et de préférence entre 1,5 et 10 % en poids.

Selon une forme de réalisation particulière de l'invention, un effet de synergie de l'action anti-oxydante est obtenu en associant le pidolate d'arginine et/ou le pidolate de lysine à au moins un dérivé phénolique.

Selon l'invention, les dérivés phénoliques sont constitués par le groupe de composés phénoliques ou polyphénoliques répondant à la définition fonctionnelle suivante :
A une concentration comprise entre 0,1 et 0,2 %, le dérivé phénolique mis en présence de 0,6 % de palmitate d'ascorbyle constitue un mélange complété par la vitamine F, qui soumis au test Rancimat à 100°C sous courant d'air de 20 ml/h, présente un temps de résistance à l'oxydation d'au moins de 200 minutes ± 10 minutes.

Parmi les dérivés phénoliques répondant à cette définition, on peut citer le tocophérol ou l'un de ses dérivés, le butylhydroxytoluène ou l'un des ses dérivés tels que le benzylidène camphre butylhydroxytoluène et les esters ou amides de l'acide caféique.

Par l'expression "tocophérol", on entend non seulement l' α-tocophérol mais également le β, γ, ou δ-tocophérol ainsi que leurs mélanges. Parmi les dérivés de tocophérol, on peut citer les précurseurs du tocophérol notamment les esters de tocophérol tels que l'acétate de tocophérol et le nicotinate de tocophérol.

Parmi les esters de l'acide caféique, on peut notamment citer les alkyl esters tels que les méthyl, éthyl ou butyl esters et le phytol ester. Parmi les amides de l'acide caféique, on peut notamment citer les N-alkylamides tels que le N-octyl amide.

Comme ceci sera rapporté ci-après, les études réalisées ont permis de mettre en évidence un important effet de synergie lorsque le pidolate d'arginine ou le pidolate de lysine était associé à un dérivé phénolique, en particulier à du tocophérol.

Selon cette forme de réalisation de l'invention, le dérivé phénolique est généralement présent à une concentration comprise entre 0,005 et 5 % en poids et de préférence entre 0,1 et 2 % en poids.

La concentration en pidolate de lysine et/ou d'arginine est généralement comprise entre 0,1 et 20 % par rapport au poids total de la composition.

L'efficacité antioxydante a été démontrée par la méthode d'oxydation accelérée de la vitamine F qui est une substance particulièrement sensible à l'oxydation.

Pour l'étude, on utilise le dispositif automatique "Rancimat" de la Société METROHM (A. Seher et al, Fette, Seifen, Anstrichmittel 88(1)1-6 1986).

On prépare des mélanges dans la vitamine F avec différentes quantités de tocophérol seul, de pidolate de lysine et de pidolate d'arginine ainsi que des systèmes binaires d'un tocophérol et d'un pidolate de lysine d'une part, et d'un tocophérol et d'un pidolate d'arginine d'autre part, ces mélanges binaires ayant été étudiés à différentes concentrations.

On porte chaque échantillon à 100°C, sous un barbotage d'air (20 litres/h). On suit alors en continu la concentration en acides volatils résultant de la dégradation des hydroperoxydes et des aldéhydes de vitamine F, dans une cellule remplie d'eau dans laquelle on plonge une électrode en platine. Cette électrode mesure, en fonction du temps, l'augmentation de la conductivité provoquée par l'augmentation de la concentration d'acides volatils. Le temps d'induction sera déterminé par l'intersection des deux asymptotes de la courbe d'oxydation exponentielle obtenue.

Ce temps correspond au temps de latence précédant l'auto-oxydation de la vitamine F. Plus ce temps de latence est long, meilleure est la résistance de la vitamine F à l'auto-oxydation.

Les résultats obtenus sont rassemblés dans le tableau suivant.

| Tocophérol | Pidolate de lysine | Pidolate d'arginine | Temps d'induction en mn |
|---|---|---|---|
| 0,1 % | - | - | 42 |
| - | 0,75 % (2,6 m.mole) | - | 126 |
| - | - | 0,83% (2,7 m.mole) | 54 |
| 0,1 % | 0,75% (2,6 m.mole) | - | 963 |
| 0,1 % | 1,5 % (5,2 m.mole) | - | 1371 |
| 0,1 % | - | 0,83% (2,7 m.mole) | 1845 |
| 0,1 % | - | 1,66% (5,4 m.mole) | 2844 |
| m.mole = millimole | | | |

Ces résultats montrent clairement que les systèmes binaires présentent une excellente activité antioxydante.

L'invention a également pour objet des compositions contenant des corps gras, celles-ci étant notamment des compositions alimentaires, des compositions cosmétiques ou dermo-pharmaceutiques.

Les corps gras présents dans les compositions de l'invention sont par exemple des corps gras d'origine animale telle que la cétine (blanc de baleine), la cire d'abeille, la lanoline, le perhydrosqualène, l'huile de tortue, etc ; des corps gras végétaux sous forme d'huiles, de graisses ou de cires tels que l'huile d'amande douce, l'huile d'avocat, l'huile d'olive, l'huile de sésame, l'huile de macadamia ; les huiles de coprah ou de palmiste hydrogénées, le beurre de cacao, la cire de Carnauba, la cire de Montana ; ainsi que des huiles synthétiques constituées par des esters et/ou éthers de glycérol ou de glycol tels que par exemple ceux décrits dans les brevets français n° 75.24656, 75.24657, et 75.24658.

En plus des corps gras plus ou moins oxydables, les compositions cosmétiques ou dermo-pharmaceutiques peuvent contenir des produits sensibles à l'oxydation tels que par exemple de la vitamine F ou du β-carotène.

Les compositions selon l'invention se présentent sous la forme de solutions huileuses, d'émulsions eau-dans-l'huile ou huile-dans-l'eau, de produits solides éventuellement anhydres, de lotions ou de microdispersions, de dispersions vésiculaires, les lipides constitutifs des vésicules pouvant être du type ionique ou non ionique ou bien un mélange de ceux-ci. Elles constituent également des laits pour les soins de la peau, des crèmes (crèmes pour le visage, pour les mains, pour le corps, crèmes antisolaires, crèmes démaquillantes, crèmes fonds de teint), des fonds de teint fluides, des laits démaquillants, des laits antisolaires, des huiles pour le bain, des rouges à lèvres, des fards à paupières, des sticks déodorants, etc ...

Pour l'application par voie topique, les compositions pharmaceutiques selon l'invention comprennent des véhicules et ingrédients nécessaires pour permettre de présenter la composition par exemple sous la forme d'onguents, de crèmes, de laits, de pommades et de solutions huileuses.

Selon une forme de réalisation préférée, les compositions cosmétiques ou dermo-pharmaceutiques se présentent sous une forme destinée à être appliquée par voie topique, en particulier de crèmes destinées à la protection de l'oxydation des lipides de la peau.

Dans les compositions selon l'invention, l'antioxydant tel que défini ci-dessus est généralement présent de sorte que l'on ait les proportions suivantes par rapport au poids total de la composition :

| | |
|---|---|
| Pidolate d'arginine ou de lysine | 0,1 à 20 % |
| Dérivé phénolique | 0,005 à 5 % |

Les compositions selon l'invention peuvent en outre contenir des composés actifs ou des ingrédients utilisés de façon usuelle dans les compositions mentionnées ci-dessus, tels que des agents tensio-actifs, des colorants, des parfums, des produits astringents, des produits absorbant l'ultraviolet, des solvants organiques. Ces compositions sont préparées selon les méthodes usuelles.

On va maintenant donner à titre d'illustration plusieurs exemples de compositions selon l'invention.

### EXEMPLE 1 : Crème hydratante

| | % |
|---|---|
| Pidolate de lysine | 5,0 |
| Lanolate de Mg | 3,0 |
| Alcool de lanoline | 5,0 |
| Huile de vaseline | 27,0 |
| Vaseline | 15,0 |
| Parahydroxybenzoate de méthyle | 0,2 |
| Parahydroxybenzoate de propyle | 0,1 |
| Eau déminéralisée qsp | 100,0 |

### EXEMPLE 2 : Crème protectrice de jour

| | % |
|---|---|
| Pidolate d'arginine | 10,0 |
| Stéarate de glycérol auto-émulsionnable | 3,0 |
| Alcool cétylique | 0,5 |
| Alcool stéarylique | 0,5 |
| Huile de vaseline | 12,0 |
| Huile de sésame | 10,0 |
| Acide stéarique | 3 |
| Parahydroxybenzoate de méthyle | 0,2 |
| Parahydroxybenzoate de propyle | 0,1 |
| Parfum | 0,3 |
| Eau déminéralisée qsp | 100 |

### EXEMPLE 3 : Crème protectrice hydratante

| | % |
|---|---|
| Pidolate de lysine | 10,0 |
| Tocophérols | 1,0 |
| Vitamine E Acétate | 1,0 |
| Stéarate de glycérol | 3,0 |
| Alcool stéarylique | 0,5 |
| Acide stéarique | 2,0 |
| Perhydrosqualène | 12,0 |
| Huile de silicone volatil | 5,0 |
| Parahydroxybenzoate de méthyle | 0,2 |
| Parahydroxybenzoate de propyle | 0,1 |
| Parfum | 0,3 |
| Eau déminéralisée qsp | 100,0 |

### EXEMPLE 4 : Crème protectrice pour les mains

| | % |
|---|---|
| Pidolate d'arginine | 1,5 |
| Caféate de méthyle | 0,4 |
| Tween 60 (Monostéarate de sorbitan polyoxyéthyléné à 20 moles d'oxyde d'éthylène) | 2,0 |
| Alcool cétylique | 1,0 |
| Myristate d'isopropyle | 3,0 |
| Huile de vaseline | 7,0 |
| Huile de silicone volatil | 7,0 |
| Parahydroxybenzoate de méthyle | 0,2 |
| Parahydroxybenzoate de propyle | 0,1 |
| Eau déminéralisée qsp | 100,0 |

### EXEMPLE 5 : Dispersion vésiculaire

| | % |
|---|---|
| Pidolate de lysine | 1,0 |
| α-tocophérol | 0,05 |
| Lécithine de soja hydrogénée | 1,8 |
| Cholestérol | 0,9 |
| Lipacide palmitoyl collagénique | 0,3 |
| Glycérine | 3,0 |
| Huile de macadamia | 15,0 |
| Huile de silicone volatil | 10,0 |
| Polymère carboxyvinylique vendu sous la dénomination de "CARBOPOL 940" par la Société GOODRICH | 0,6 |
| Parahydroxybenzoate de méthyle | 0,2 |
| Triéthanolamine | qs pH = 6 |
| Eau déminéralisée qsp | 100,0 |

## Revendications

1. Utilisation du pyrrolidone carboxylate de lysine (pidolate de lysine) et/ou du pyrrolidone carboxylate d'arginine (pidolate d'arginine) comme substance antioxydante dans la préparation d'une composition cosmétique ou pharmaceutique, destinée au traitement de la peau et en particulier de son vieillissement.

2. Utilisation selon la revendication 1, caractérisée par le fait que le pidolate de lysine et/ou d'arginine est présent dans la composition à une concentration comprise entre 0,1 et 20 % en poids.

3. Utilisation selon l'une des revendications 1 et 2, caractérisée par le fait que le pidolate de lysine et/ou d'arginine est associé à au moins un dérivé phénolique.

4. Utilisation selon la revendication 3, caractérisée par le fait que le dérivé phénolique est le tocophérol ou l'un de ses dérivés, le butylhydroxytoluène, ou l'un de ses dérivés tels que le benzylidène camphre butylhydroxytoluène et les esters ou amides de l'acide caféique.

5. Utilisation selon la revendication 4, caractérisée par le fait que le tocophérol est choisi parmi l'α-tocophérol, le β-tocophérol, le γ-tocophérol, le δ-tocophérol et leurs mélanges.

6. Utilisation selon la revendication 4, caractérisée par le fait que le dérivé de tocophérol est choisi parmi l'acétate de tocophérol et le nicotinate de tocophérol.

7. Utilisation selon les revendications 3 à 6, caractérisée par le fait que le dérivé phénolique est présent à une concentration comprise entre 0,005 et 5 % en poids.

8. Utilisation selon l'une quelconque des revendications 1 à 7, caractérisée par le fait que le pidolate d'arginine et/ou le pidolate de lysine est présent à une concentration comprise entre 0,1 et 20 % en poids.

9. Composition cosmétique ou pharmaceutique caractérisée par le fait qu'elle contient en tant que substance antioxydante l'association suivante :
| | |
|---|---|
| Pidolate d'arginine et/ou de lysine | 0,1 à 20 % |
| Dérivés(s) phénolique(s) | 0,005 à 5 % |
- les pourcentages étant exprimés en poids par rapport au poids total de la composition.

10. Composition selon la revendication 9, caractérisée par le fait qu'elle se présente sous forme d'une crème destinée à la protection de l'oxydation des lipides de la peau.

## Claims

1. Use of lysine pyrrolidonecarboxylate (lysine pidolate) and/or of arginine pyrrolidonecarboxylate (arginine pidolate) as antioxidizing substance in the preparation of a cosmetic or pharmaceutical composition, intended for treatment of the skin and in particular of its aging.

2. Use according to Claim 1, characterized in that lysine and/or arginine pidolate is present in the composition at a concentration of between 0.1 and 20% by weight.

3. Use according to one of Claims 1 and 2, characterized in that lysine and/or arginine pidolate is in combination with at least one phenol derivative.

4. Use according to Claim 3, characterized in that the phenol derivative is tocopherol or one of its derivatives, butylhydroxytoluene or one of its derivatives such as benzylidenecamphorbutylhydroxytoluene, and the esters or amides of caffeic acid.

5. Use according to Claim 4, characterized in that the tocopherol is chosen from α-tocopherol, β-tocopherol, γ-tocopherol, δ-tocopherol and their mixtures.

6. Use according to Claim 4, characterized in that the tocopherol derivative is chosen from tocopherol acetate and tocopherol nicotinate.

7. Use according to Claims 3 to 6, characterized in that the phenol derivative is present at a concentration of between 0.005 and 5% by weight.

8. Use according to any one of Claims 1 to 7, characterized in that arginine pidolate and/or lysine pidolate is present at a concentration of between 0.1 and 20% by weight.

9. Cosmetic or pharmaceutical composition, characterized in that it contains, as antioxidizing substance, the following combination:
| | |
|---|---|
| Arginine and/or lysine pidolate | 0.1 to 20% |
| Phenol derivative(s) | 0.005 to 5% |
- the percentages being expressed by weight with respect to the total weight of the composition.

10. Composition according to Claim 9, characterized in that it is provided in the form of a cream intended for protecting the lipids of the skin from oxidation.

## Patentansprüche

1. Verwendung von Lysin-5-oxo-2-pyrrolidincarboxylat (Lysinpidolat (Lysinpyroglutaminat)) und/oder Arginin-5-oxo-2-pyrrolidincarboxylat (Argininpidolat (Argininpyroglutaminat)) als Antioxidationsmittel bei der Herstellung einer kosmetischen oder pharmazeutischen Zubereitung zur Behandlung der Haut und insbesondere gegen deren Alterung.

2. Verwendung gemäß Anspruch 1, dadurch gekennzeichnet, daß das Lysin- und/oder Argininpidolat in der Zubereitung in einer Konzentration von zwischen 0,1 und 20 Gew.-% vorliegt.

3. Verwendung gemäß einem der Ansprüche 1 und 2, dadurch gekennzeichnet, daß das Lysin- und/oder Argininpidolat in Kombination mit mindestens einem Phenolderivat vorliegt.

4. Verwendung gemäß Anspruch 3, dadurch gekennzeichnet, daß es sich bei dem Phenolderivat um Tocopherol oder eines seiner Derivate, um Burylhydroxytoluol oder eines seiner Derivate, wie Kampferbenzylidenbutylhydroxytoluol, oder auch um einen Kaffeinsäureester oder -amid handelt.

5. Verwendung gemäß Anspruch 4, dadurch gekennzeichnet, daß das Tocopherol aus l'-α-Tocopherol, β-Tocopherol, γ-Tocopherol, δ-Tocopherol oder deren Gemischen ausgewählt ist.

6. Verwendung gemäß Anspruch 4, dadurch gekennzeichnet, daß das Tocopherolderivat aus Tocopherolacetat und -nikotinat ausgewählt ist.

7. Verwendung gemäß den Ansprüchen 3 - 6, dadurch gekennzeichnet, daß das Phenolderivat in einer Konzentration von 0,005 bis 5 Gew.-% vorhanden ist.

8. Verwendung gemäß einem der Ansprüche 1 bis 7, dadurch gekennzeichnet, daß das Arginin- und/oder Lysinpidolat in Konzentrationen zwischen 0,1 und 20 Gew.-% vorhanden ist.

9. Kosmetische oder pharmazeutische Zubereitung, dadurch gekennzeichnet, daß als Antioxidationsmittel das folgende Kombinationsprodukt enthält:
| | |
|---|---|
| - Arginin- und/oder Lysinpidolat | 0,1 bis 20 % |
| - Phenolderivat(e) | 0,005 bis 5 %, |
wobei die Prozentangaben in Gewichtsprozent, bezogen auf das Gesamtgewicht der Zubereitung, angegeben sind.

10. Zubereitung gemäß Anspruch 9, dadurch gekennzeichnet, daß sie in Form einer Creme vorliegt, welche die Hautlipide vor der Oxidation schützen soll.
